# EUROPÄISCHE PATENTANMELDUNG

(11) **EP 2 284 218 A1**
(43) Veröffentlichungstag der Anmeldung: **16.02.2011**
(21) Anmeldenummer: 09166463.1
(22) Anmeldetag: 27.07.2009
(51) Int. Cl.: C08K 5/45, C08K 3/22, A61L 2/08, B01J 19/12

(54) **Polymerzusammensetzung für Photobioreaktoren**

(71) Anmelder: Georg Fischer DEKA GmbH, 35232 Dautphetal-Mornshausen (DE)
(72) Erfinder: Schüssler, Stephan, 35094, Caldern (DE); Gaul, Inno, 53359 Rheinbach (DE); Kuppelmaier, Harald, 68809 Neulussheim (DE); Proper, Gerrit, 8162 RW Epe (NL)
(74) Vertreter: De Colle, Piergiacomo

(57) **Zusammenfassung**

Es wird eine Polymerzusammensetzung mit einem modifizierten Absorptions- und Transmissionsverhalten vorgeschlagen, insbesondere geeignet für dem Sonnenlicht oder geeigneten Kunstlichtquellen ausgesetzten Photoreaktoren oder Photobioreaktoren aus Kunststoffformkörpern, wobei das Polymer zusätzlich zu den herkömmlichen Standard-Additiven wahlweise eine oder eine Kombination der folgenden Substanzen aufweist: ein anorganischer oder organischer Nah-Infrarot-Absorber zur Absorption der langweiligen Strahlung, ein anorganischer oder organischer Reflektor zur Reflexion von Ultraviolettstrahlung, ein anorganischer oder organischer Reflektor zur Reflexion von sichtbarer, Nah-Infrarot- oder Infrarot-Strahlung, ein optischer Aufheller oder Fluoreszenzfarbstoff zur Umwandlung der absorbierten Ultraviolettstrahlung in sichtbares Licht oder Fluoreszenzlicht, ein photochromer Farbstoff zur lichtintensitätsabhängigen Modifizierung des Transmissionsverhaltens des Kunststoffformkörpers und ein antimikrobieller Zusatz zur Verhinderung oder Reduktion der organischen Ablagerungen in dem Photobioreaktor. Der Photobioreaktor weist eine schraubenlinienförmig ausgebildete Innenoberfläche zur effizienten Durchmischung des Reaktionsmediums auf.

## Beschreibung

Die Erfindung bezieht sich auf eine Polymerzusammensetzung mit einem modifizierten Absorptions- und Transmissionsverhalten, insbesondere geeignet für dem Sonnenlicht oder geeigneten Kunstlichtquellen ausgesetzten Photoreaktoren oder Photobioreaktoren aus Kunststoffformkörpern.

Photoreaktoren sind Reaktionsgefässe zur Durchführung von photochemischen Reaktionen. Die Reaktionsmedien sind Lösungen oder Suspensionen, die unter Einwirkung von Licht Reaktionen eingehen. Photobioreaktoren sind Reaktionsgefässe zur Durchführung von photobiologischen Reaktionen ähnlich der Photosynthese im Pflanzenreich. In diesen Photobioreaktoren werden beispielsweise Mikroalgen zur Herstellung von Biotreibstoffen, beispielsweise Biodiesel als Form erneuerbarer Energie, verwendet. Die Anwendung von Photobioreaktoren in der Zucht von Mikroalgen hat auch eine wachsende Bedeutung in der Herstellung von Algenkonzentraten mit anderen Anwendungsbereichen, wie beispielsweise die Fischzucht, die Herstellung von Nahrungsmittelzusatzstoffen oder als Binder oder Neutralisator von Kohlendioxid aus Abgasen der thermischen Kraftwerken.

An die Wandmaterialien des Reaktionsgefässes werden hohe Anforderungen gestellt. Der Werkstoff für die Wände muss eine möglichst hohe Beständigkeit gegen Ultraviolettstrahlung aufweisen. Die UV-Strahlung kann schädlich sein für das Reaktionsmedium und muss deshalb entweder abgehalten oder reflektiert werden, oder in für das Reaktionsmedium geeigneter Strahlung (sichtbares Licht der Wellenlänge 400 bis 700 nm) umgewandelt werden.

Der Werkstoff muss eine möglichst optimale Transparenz für die geeignete Strahlung aufweisen. Die im Sonnenlicht enthaltene Nah-Infrarotstrahlung (NIR) ist massgeblich verantwortlich für die Erwärmung des Photobioreaktors und der Algensuspension. Da das Algenwachstum nur in einem bestimmten moderaten Temperaturbereich optimal verläuft, muss der Reaktor temperiert werden. Das Konzept zur Temperierung hat einen entscheidenden Einfluss auf die bauliche Ausführung des Photobioreaktors und seine Effizienz.

Weiterhin ist es Ziel einer optimalen Photobioreaktoranordnung, dass die pro Standflächeneinheit eingestrahlte für das Algenwachstum nutzbare Strahlung möglichst vollständig für das Algenwachstum nutzbar gemacht wird. Eine Maximierung der Photobioreaktorfläche pro Standflächeneinheit ist daher ein wichtiges Ziel in der Optimierung der Effizienz von Photobioreaktoren. Eine intelligente Schichtung von Photobioreaktoren bei gleichzeitiger wirkungsvoller Verteilung der auftreffenden Strahlung auf eine möglichst grosse Reaktorfläche muss das Ziel sein.

Weiterhin muss der Werkstoff eine möglichst hohe mechanische Stabilität aufweisen. Die transparente Wand des Reaktors darf nicht durch Ablagerungen verschmutzt werden, d.h. auf der Innenseite des Reaktors müssen Ablagerungen verhindert werden. Weil für die Durchführung der photobiologischen Reaktionen sehr grosse Reaktoren, sprich sehr lange Rohre erforderlich sind, spielen auch das Gewicht und der Preis des Reaktionsgefässes eine grosse Rolle.

Aus der EP 1127612 ist ein solarer Photoreaktor bekannt. Das Reaktionsgefäss besteht aus einem doppelwandigen Rohrsystem, bei dem das Reaktionsmedium im Spalt zwischen den zwei Rohren gefördert wird. Das Reaktionsmedium wird von aussen und von innen der solaren Strahlungsenergie oder einer geeigneten Kunstlichtquelle ausgesetzt. Für das Reaktionsgefäss werden für die Sonnenstrahlung transparente Rohre aus Glas oder Kunststoff vorgeschlagen.

Ausgehend von diesem Stand der Technik ist es Aufgabe der Erfindung, eine Polymerzusammensetzung für Photoreaktoren, insbesondere für Photobioreaktoren anzugeben, die das Polymermaterial für die Wand des Reaktors an die Prozessbedingungen der Photosynthese optimal anpassbar macht, ein möglichst niedriges Gewicht aufweist und eine möglichst lange Lebensdauer garantiert.

Diese Aufgabe wird gelöst durch eine Polymerzusammensetzung mit einem modifizierten Absorptions- und Transmissionsverhalten, insbesondere geeignet für dem Sonnenlicht oder geeigneten Kunstlichtquellen ausgesetzten Photoreaktoren oder Photobioreaktoren aus Kunststoffformkörpern, wobei das Polymer zusätzlich zu den herkömmlichen Standard-Additiven wahlweise eine oder eine Kombination der folgenden Substanzen aufweist: ein anorganischer oder organischer Nah-Infrarot-Absorber zur Absorption der langwelligen Strahlung, ein anorganischer oder organischer Reflektor zur Reflexion von Ultraviolettstrahlung, ein anorganischer oder organischer Reflektor zur Reflexion von sichtbarer, Nah-Infrarot- oder Infrarot-Strahlung, ein optischer Aufheller oder Fluoreszenzfarbstoff zur Umwandlung der absorbierten Ultraviolettstrahlung in sichtbares Licht oder Fluoreszenzlicht, ein photochromer Farbstoff zur lichtintensitätsabhängigen Modifizierung des Transmissionsverhaltens des Kunststoffformkörpers und ein antimikrobieller Zusatz zur Verhinderung oder Reduktion der organischen Ablagerungen in dem Photobioreaktor.

Bevorzugte Weiterbildungen der Erfindung ergeben sich aus den abhängigen Ansprüchen.

Es ist von Vorteil, dass das Reaktionsmedium im Photobioreaktor gegen Ultraviolettstrahlung geschützt wird. Dies wird dadurch erreicht, dass der Reflektor Titandioxidpartikel mit Korngrössen im sub-Mikrometer- oder Nanometerbereich aufweist. Nanoskalige Titandioxidpartikel können in entsprechender Grösse und bei optimaler Verteilung selektiv und dauerhaft wirksam als UV-Absorber eingesetzt werden. Eine Kombination vom nanoskaligen Titandioxid mit submikroskaligen Titandioxid hat zur Folge, dass mit möglichst wenig Zusatzmaterial ohne den Einsatz von konventionellem UV-Absorber sowohl eine optimale Reflexion von UV-Strahlung als auch ein breitbandiger Schutz gegen sichtbares und NIR-Licht erreicht wird.

Es ist auch von Vorteil, dass das Wärmemanagement im Reaktor gesteuert werden kann. Dies wird dadurch erreicht, dass der Nah-Infrarot-Absorber vorzugsweise ein anorganisches auf seltenen Erdmetallen basierendes Pigment aufweist. Der NIR-Absorber kann entweder homogen über die gesamte Wandstärke des Rohres verteilt angeordnet sein, oder bei einem co-extrudierten Rohr nur in der äusseren Schicht.

Es ist weiter auch von Vorteil, dass die schädliche UV-Strahlung in unschädliches blaues oder grünes Licht umgewandelt wird. Dies wird dadurch erreicht, dass der optische Aufheller vorzugsweise Verbindungen auf Thiophen-Benzoxazol-Basis aufweist.

Es ist auch von Vorteil, dass im Photobioreaktor die optimale Lichtintensität zur Verfügung gestellt wird. Dies wird dadurch erreicht, dass der photochrome Farbstoff vorzugsweise Spiro-Naphthoxazine oder Naphthopyrane aufweist.

Es ist auch von Vorteil, dass auf der Innenoberfläche des Reaktors Ablagerungen durch Algenwachstum verhindert oder reduziert werden. Dies wird dadurch erreicht, dass der antimikrobielle Zusatz vorzugsweise Verbindungen auf Carbamat- oder Silberbasis aufweist. Dies wird auch dadurch erreicht, dass die Rohrwand eine totraumfreie Innenfläche aufweist. Dies wird weiterhin auch dadurch erreicht, dass die Rohrwand auf der Innenseite als ein statischer Mischer ausgebildet ist. Dieser statische Mischer unterstützt auch die gleichmässige Bestrahlung der Algensuspension und fördert eine gleichmässige Temperaturverteilung im Reaktionsmedium.

Das Wandmaterial wird durch die neue Polymerzusammensetzung so modifiziert, dass im Photobioreaktor über die gesamte Standzeit optimale Bedingungen für das Wachstum der Mikroalgen und für die effiziente Produktion von Biomasse oder Biodiesel angeboten werden. Optimal heisst hier, dass die richtigen Wellenlängen aus dem Strahlungsspektrum in der richtigen Intensität durchgelassen werden, dass die schädlichen Wellenlängen reflektiert oder in für das Algenwachstum unschädliche Strahlung umgewandelt werden und dass die Wandinnenseite gegen Ablagerungen geschützt wird. Das Wandmaterial erhält für den Betrieb im Photobioreaktor optimale Eigenschaften, die über die gesamte Standzeit des Reaktors konstant bleiben, d.h. die Lichtdurchlässigkeit des Wandmaterials bleibt konstant und die Wand wird nicht matt.

Durch die Reflektoreigenschaften der neuen Polymerzusammensetzung leistet die so modifizierte Wand des Photobioreaktors einen effektiven Beitrag zur Lichtverdünnung. Die reflektierte Strahlung kann auf benachbarte Photobioreaktorflächen gespiegelt werden. Die pro Standflächeneinheit auftreffende Lichtmenge wird so auf eine grössere Fläche an bestrahltem Photobioreaktor verteilt.

Ausführungsbeispiele der Erfindung werden anhand der Figuren beschrieben. Es zeigen:
Figur 1 einen Schnitt durch einem erfindungsgemässen Rohr für einen Photobioreaktor,
Figur 2 einen weiteren Schnitt durch einem Rohr für einen Photobioreaktor,
Figur 3 eine Zusammenstellung der Messresultate zum Wärmemanagement im Photobioreaktor mit einer erfindungsgemässen Polymerzusammensetzung im Vergleich zu einem konventionellen Polymer,
Figur 4 eine Darstellung der Wirkung des Zusatzes zur Umwandlung der UV-Strahlung in sichtbarem Licht im Vergleich zu einem konventionellen Polymer,
Figur 5 eine Darstellung des Transmissionsverhaltens in Abhängigkeit der Wellenlänge von konventionellem Polymermaterial im Vergleich zur erfindungsgemässen Polymerzusammensetzung mit einem geeigneten Titandioxidpartikelzusatz und
Figur 6 eine Darstellung des Transmissionsverhaltens in Abhängigkeit der Wellenlänge von konventionellem Polymermaterial im Vergleich zur erfindungsgemässen Polymerzusammensetzung mit einem geeigneten photochromen Additivzusatz.

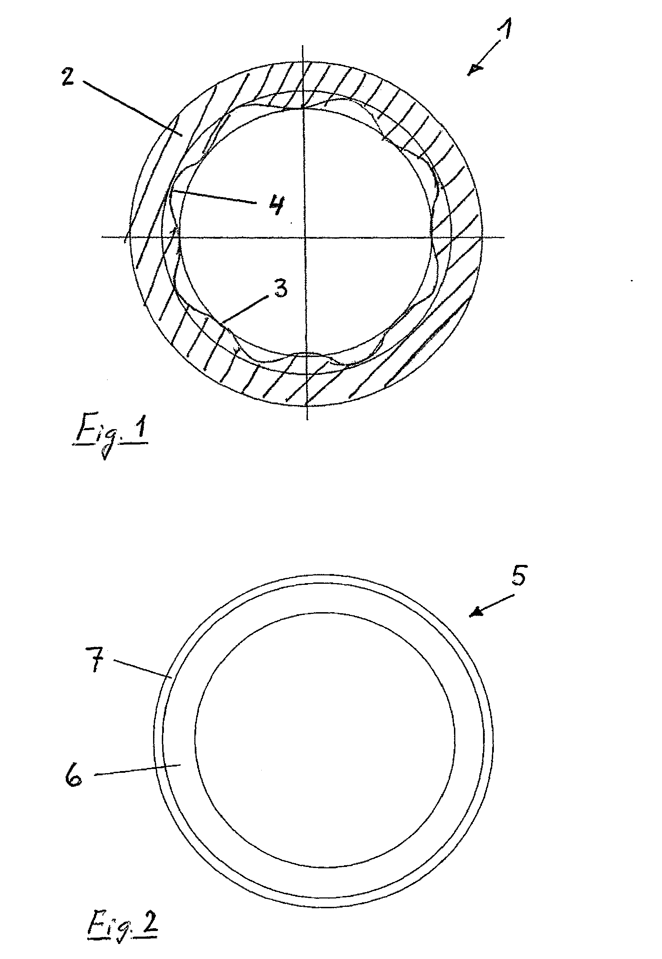

In Figur 1 ist ein PVC-Rohr 1 geschnitten dargestellt. Das PVC-Rohr 1 wird als ein Kunststoffformkörper durch Extrusion hergestellt und weist auf der Innenseite eine Rohrwand 2 mit einer schraubenlinienförmig ausgebildeten Innenfläche 3 auf. Hierdurch wird die Strömung des Reaktionsmediums wie in einem statischen Mischer beeinflusst. Durch die spiralförmige Rillen 4 oder Strukturierung der Innenoberfläche 3 wird im Rohrreaktor auch bei kleineren Strömungsgeschwindigkeiten eine effiziente Mischung des Reaktionsmediums ohne grösseren Druckverlust möglich. Die Innenoberfläche 3 weist keine Toträume auf, d.h. es gibt keine Bereiche, wo die Strömungsgeschwindigkeit lokal so reduziert wird, dass Ablagerungen ausfallen. Die Innenoberfläche 3 ist noch einfach genug zu reinigen und es erfolgen durch die Struktur keine Streuung und Einkoppelverluste für die Strahlung zum Reaktionsmedium.

Statt transparentem PVC kann jeder andere Polymerwerkstoff eingesetzt werden, die im Absorptions- und Transmissionsverhalten für die Prozesse im Photobioreaktor modifiziert werden können. Als Beispiele für geeignete Polymere kommen neben transparentes Polyvinylchlorid, Polycarbonat, Polymethylmetacrylat, Polyolefin, Polystyrol, Polyethylenterephthalat, Polybutylenterephthalat oder Kombinationen, teil- oder vollfluorierte Polymere, wie beispielsweise Polyvinylidenfluorid oder Perfluoralkoxalkan, Copolymeren oder Legierungen hieraus in Frage.

Die Figur 2 zeigt einen weiteren Schnitt durch ein Rohr 5 eines Photobioreaktors. Das Rohr 5 aus Figur 2 wird durch Co-extrusion hergestellt. Die Rohrwand ist aus einer dickeren tragenden Innenschicht 6 und einer dünneren funktionalen Aussenschicht 7 aufgebaut. Die Innenschicht 6 kann mit einem antimikrobiellen Zusatz und mit einem optischen Aufheller oder Fluoreszenzfarbstoff ausgerüstet werden. Die Aussenschicht 7 ist vorzugsweise weniger als 1 mm stark und wird zur Modifikation des Absorptions- und Transmissionsverhalten ausgerüstet. Die Aussenschicht 7 enthält die Kombination vom nanoskaligen Titandioxid mit submikroskaligen Titandioxid und den Nah-Infrarot Absorber, vorzugsweise ein anorganisches auf seltenen Erdmetallen basierendes Pigment.

Durch die Verlagerung des Wellenlängen-Managements in der dünneren Aussenschicht 7 werden folgende Vorteile erreicht: Die Haupt- bzw. Innenschicht 6 wird als thermischer Isolator genutzt. Dadurch wird der pro Flächeneinheit für das Erreichen eines bestimmten Kühleffektes notwendige absolute Zusatz des NIR-Absorbers in der Aussenschicht 7 reduziert. Die Lebensdauer des optischen Aufhellers in der Innen- und/oder Aussenschicht 6, 7 wird deutlich verlängert, da die UV-Einstrahlung deutlich reduziert ist bzw. gesteuert werden kann. In der Innenschicht 6 sind nur geringe Gehalte an konventionellen UV-Schutzadditiven erforderlich. Durch den Schichtaufbau wird in der Aussenschicht 7 zudem eine Totalreflexion der ausgefilterten Wellen möglich. Durch die gezielte Aufteilung der Additiven auf Innen- und Aussenschicht 6, 7 werden zusätzlich destruktive Wechselwirkungen zwischen den verschieden Additiven verhindert, was zu einer längeren Lebensdauer des Verbundmaterials führt.

Anstelle eines co-extrudierten Kunststoffrohres kann auch ein bestehendes Rohrmaterial mit der erfindungsgemässen Polymerzusammensetzung an der Aussenseite beschichtet, kaschiert oder mit einer dünnen Folie laminiert werden. Denkbar ist auch, dass bestehende Reaktorrohre aus Glas oder Keramik mit einer solchen Folie bedeckt werden.

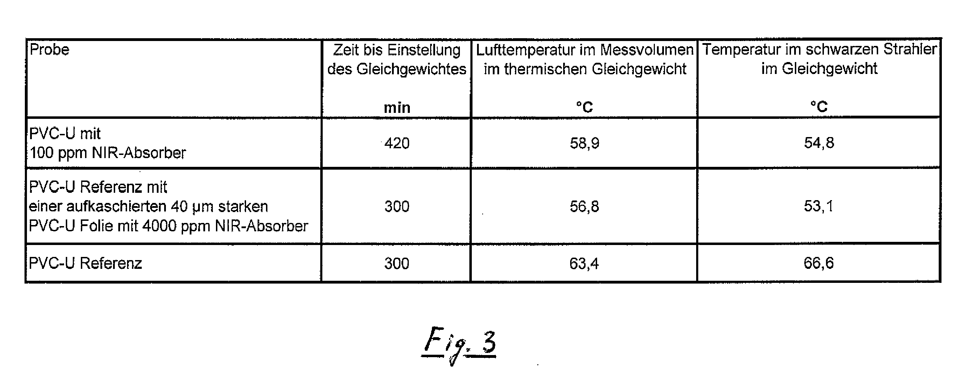

Figur 3 zeigt in einer Tabelle eine Zusammenstellung der Messresultate zum Wärmemanagement im Photobioreaktor mit einer erfindungsgemässen Polymerzusammensetzung im Vergleich zu einem konventionellen Polymer. Als Probekörper wurden neben einer unbehandelten transparenten PVC-U-Platte mit einer Dicke von 3 mm eine solche Platte mit 100 ppm NIR-Absorber und ein Verbund aus einer unbehandelten Platte mit einer aufkaschierten 40 µm starken PVC-U-Folie mit 4000 ppm des gleichen NIR-Absorbers mit einander verglichen. Unterhalb der Platten wurde jeweils in einem ruhenden Luftvolumen die Zeit bis zur Einstellung des Gleichgewichtes, die Temperatur der Luft und die Temperatur eines schwarzen Strahlers im Gleichgewichtszustand gemessen. Die Temperatur am schwarzen Strahler kann als Mass für einen reduzierten Wärmfluss auf das im Rohr transportierte Medium gesehen werden und demonstriert somit die Effizienz des NIR-Absorbers.

Die Messdaten zeigen, dass selbst bei einer homogen mit 100 ppm NIR-Absorber pigmentierten Wand eine deutliche Reduzierung der Temperatur erreicht wird. Wenn jedoch der NIR-Absorber gezielt in der dünneren Aussenschicht zugesetzt wird, kann nicht nur der Verbrauch an NIR-Absorber insgesamt deutlich reduziert werden, sondern eine weitere Reduzierung der Temperatur erreicht werden. In einem Verbund wird die innen liegende Schicht als thermischer Isolator genutzt. Die NIR-Barriere wird auf die Aussenschicht verlegt. Als NIR-Absorber wird beispielsweise Lumogen von BASF zugesetzt.

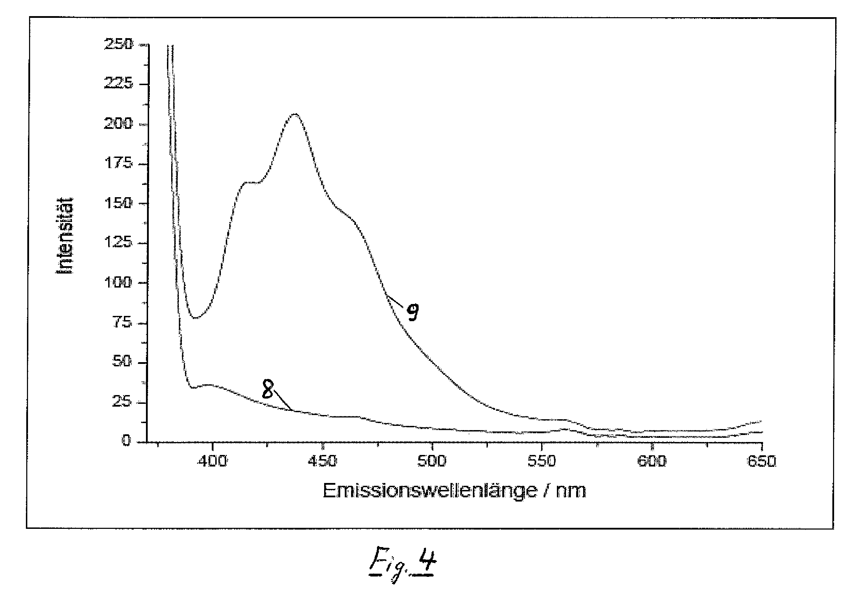

In Figur 4 wird die Intensität in Abhängigkeit der Wellenlänge eines Referenzmusters (Kurve 8) und einer Probe (Kurve 9) mit einem optischen Aufheller dargestellt. Hier ist die Wirkung des Zusatzes zur Umwandlung der UV-Strahlung in sichtbarem Licht im Vergleich zu einem konventionellen Polymer dargestellt. Als Probekörper wurden 0,3 mm dicke PVC-U-Platten gepresst. In einem Probekörper wurden 100 ppm eines UV-aktiven Fluoreszenzfarbstoffes zugesetzt. An beiden Platten wurde das Emissionspektrum nach Anregung mit Laserstrahlung im UV-Bereich aufgezeichnet.

Aus dem Vergleich wird sichtbar, dass die Fluoreszenzstrahlung genau mit dem für das Algenwachstum relevanten Lichtwellenbereich von 400 bis 700 nm zusammenfällt. Als Fluoreszenzfarbstoff wird beispielsweise Uvitex OB von CIBA zugesetzt.

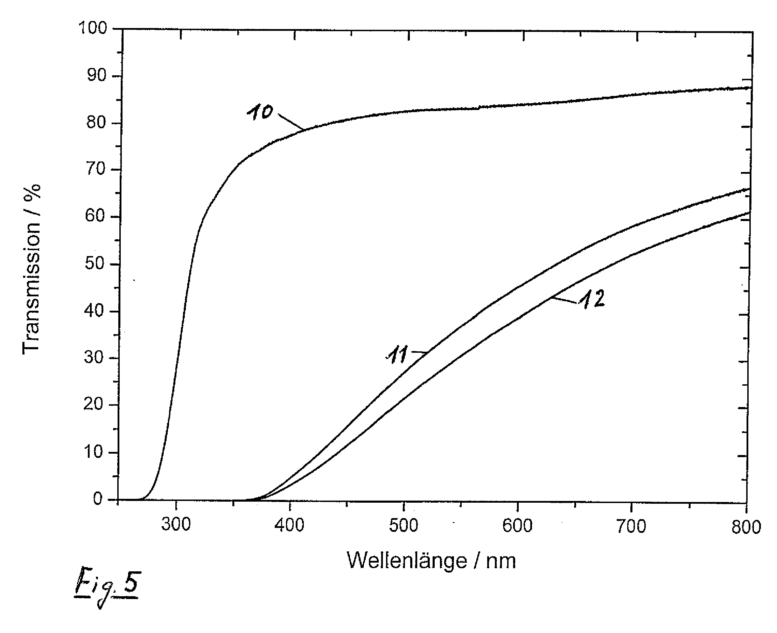

In Figur 5 ist das Transmissionsverhalten in Abhängigkeit der Wellenlänge von konventionellem Polymermaterial (Kurve 10) im Vergleich zur erfindungsgemässen Polymerzusammensetzung mit einem geeigneten Titandioxidpartikelzusatz dargestellt. Als Probekörper wurden wiederum 0,3 mm dicke PVC-U-Platten hergestellt. In einem Probekörper (Kurve 11) wurden 0.5 Gew.% nanoskaliges Titandioxid zugesetzt. In einem weiteren Probekörper (Kurve 12) wurden 0.5 Gew.% nanoskaliges Titandioxid und 0.003 Gew.% submikroskaliges Titandioxid zugesetzt.

Aus dem Vergleich wird sichtbar, dass mit dem Zusatz des nanoskaligen Titandioxids alleine ein sehr effizienter UV-Schutz ohne Einsatz vom konventionellen UV-Absorber erreicht wird. Wenn zusätzlich noch eine sehr geringe Menge an submikroskaligem Titandioxid zugesetzt wird, so wird ein breitbandiger Reflektorschutz für sichtbare und NIR-Wellenlängen erreicht.

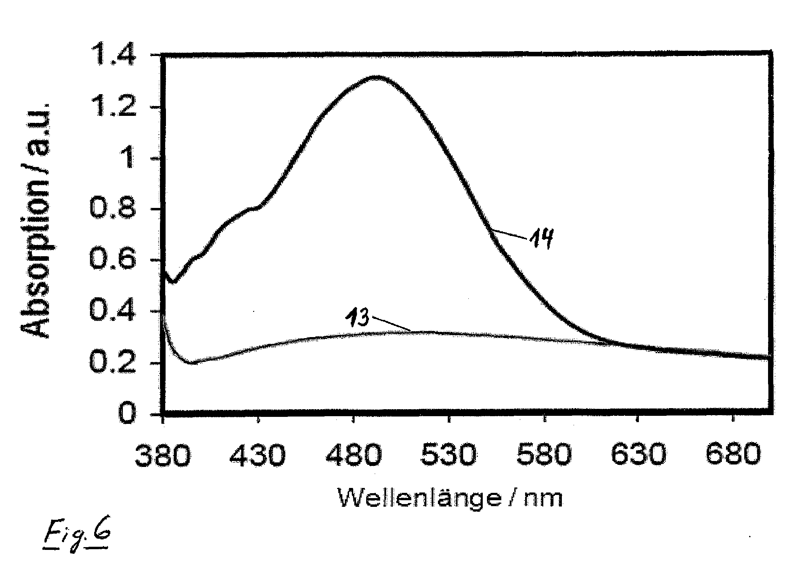

In Figur 6 ist das Transmissionsverhalten in Abhängigkeit der Wellenlänge von konventionellem Polymermaterial (Kurve 13) im vergleich zur erfindungsgemässen Polymerzusammensetzung (Kurve 14) mit einem geeigneten photochromen Farbstoffpartikelzusatz dargestellt. Als Probekörper wurden wiederum 0,3 mm dicke transparente PVC-U-Platten hergestellt. In einem Probekörper (Kurve 14) wurden 300 ppm photochromer Farbstoff zugesetzt und fünf Minuten mit einer Halogenlampe bestrahlt. Als photochromer Farbstoff wird beispielsweise Reversacol von James Robinson eingesetzt.

Aus dem Spektrum ist ersichtlich, dass durch die Einwirkung von UV-Strahlung der photochrome Farbstoff in seine gefärbte Form überführt wird und genau in dem Bereich absorbiert, der auch für das Alögenwachstum relevant ist. Dieser Vorgang ist reversibel. Hierdurch kann also eine Lichtintensitätsabhängige Steuerung des Transmissionsverhaltens der Polymerzusammensetzung erreicht werden.

Durch vierwöchige Einlagerungsversuche von Mustern aus transparenten PVC-U Rohren der Dimension 63 x 3,0 mm basierend auf einer konventionell UVstabilisierten Rezeptur mit 0,1 % Carbamat-basierenden Biozidzusatz (Fungitrol von ISP) in einer Algenlösung unter Standard-Betriebsbedingungen (T = 25°C, p < 1 bar) konnte gegenüber einer nicht Biozid-additivierten Rohrprobe eine deutliche Reduktion des Algenbewuchses festgestellt werden. Der Effekt der Reduktion des Bewuchses wurde auf ca. 50 % geschätzt.

Die hier beschriebene Anwendung der Polymerzusammensetzung im Photobioreaktor kann auch in anderen Photoreaktoren angewendet werden. Die Rohre werden vorzugsweise mit sogenannten Tridamp-Verbindern verbunden. Triclampverbindungen sind leicht, platzsparend und trotzdem leicht und schnell lösbar. Das Rohrende wird hierzu auf der Baustelle mit einer Bundbuchse mit einer abgewinkelten Flanke verklebt oder verschweisst. Diese Verbindungsart ist in der Wartung zeitsparend und flexibel. Statt Rohre können auch Platten oder andere Kunststoffformkörper mit der neuen Polymerzusammensetzung hergestellt werden.

## Patentansprüche

1. Polymerzusammensetzung mit einem modifizierten Absorptions- und Transmissionsverhalten, insbesondere geeignet für dem Sonnenlicht oder geeigneten Kunstlichtquellen ausgesetzten Photobioreaktoren aus Kunststoffformkörpern, **dadurch gekennzeichnet, dass** das Polymer zusätzlich zu den herkömmlichen Standard-Additiven wahlweise eine oder eine Kombination der folgenden Substanzen aufweist:
- ein anorganischer oder organischer Nah-Infrarot-Absorber zur Absorption der langwelligen Strahlung,
- ein anorganischer oder organischer Reflektor zur Reflexion von Ultraviolettstrahlung,
- ein anorganischer oder organischer Reflektor zur Reflexion von sichtbarer, Nah-Infrarot- oder Infrarot-Strahlung,
- ein optischer Aufheller oder Fluoreszenzfarbstoff zur Umwandlung der absorbierten Ultraviolettstrahlung in sichtbares Licht oder Fluoreszenzlicht,
- ein photochromer Farbstoff zur lichtintensitätsabhängigen Modifizierung des Transmissionsverhaltens des Kunststoffformkörpers und
- ein antimikrobieller Zusatz zur Verhinderung oder Reduktion der organischen Ablagerungen in dem Photobioreaktor.

2. Polymerzusammensetzung nach dem Anspruch 1, **dadurch gekennzeichnet, dass** der Reflektor Titandioxidpartikel mit Korngrössen im sub-Mikrometer- oder Nanometerbereich aufweist.

3. Polymerzusammensetzung nach mindestens einem der Ansprüche 1 oder 2, **dadurch gekennzeichnet, dass** der Nah-Infrarot-Absorber vorzugsweise ein anorganisches auf seltenen Erdmetallen basierendes Pigment aufweist.

4. Polymerzusammensetzung nach mindestens einem der Ansprüche 1 bis 3, **dadurch gekennzeichnet, dass** der optische Aufheller vorzugsweise Verbindungen auf Thiophen-Benzoxazol-Basis aufweist.

5. Polymerzusammensetzung nach mindestens einem der Ansprüche 1 bis 4, **dadurch gekennzeichnet, dass** der photochrome Farbstoff vorzugsweise Spiro-Naphthoxazine oder Naphthopyrane aufweist.

6. Polymerzusammensetzung nach mindestens einem der Ansprüche 1 bis 5, **dadurch gekennzeichnet, dass** der antimikrobielle Zusatz vorzugsweise Verbindungen auf Carbamat- oder Silberbasis aufweist.

7. Polymerzusammensetzung nach mindestens einem der Ansprüche 1 bis 6, **dadurch gekennzeichnet, dass** das Polymer amorph oder teilkristallin ausgebildet ist.

8. Polymerzusammensetzung nach mindestens einem der Ansprüche 1 bis 7, **dadurch gekennzeichnet, dass** als Polymer wahlweise transparentes Polyvinylchlorid, Polycarbonat, Polymethylmetacrylat, Polyolefin, Polystyrol, Polyethylenterephthalat, Polybutylenterephthalat oder Kombinationen, teil- oder vollfluorierte Polymere, wie beispielsweise Polyvinylidenfluorid oder Perfluoralkoxalkan, Copolymeren oder Legierungen hieraus verwendet wird.

9. Verwendung der Polymerzusammensetzung nach mindestens einem der Ansprüche 1 bis 8 in einem als Photobioreaktor einsetzbaren Kunststoffformkörper, **dadurch gekennzeichnet, dass** die Wand des Photobioreaktors rohrförmig oder plattenförmig ausgebildet ist.

10. Verwendung der Polymerzusammensetzung nach mindestens einem der Ansprüche 1 bis 9 in einem Kunststoffformkörper, **dadurch gekennzeichnet, dass** der Kunststoffformkörper aus verschiedenen Schichten, beispielsweise durch Co-extrusion, Kaschierung oder Beschichtung ausgebildet ist und dass die Zusätze in den verschiedenen Schichten unterschiedliche Konzentrationen aufweisen.

11. Verwendung der Polymerzusammensetzung nach mindestens einem der Ansprüche 1 bis 10 in einem Photobioreaktor, **dadurch gekennzeichnet, dass** der rohrförmig extrudierte Kunststoffformkörper eine Rohrwand mit einer schraubenlinienförmig ausgebildeten Innenfläche aufweist.

12. Verwendung der Polymerzusammensetzung nach mindestens einem der Ansprüche 1 bis 11 in einem Photobioreaktor, **dadurch gekennzeichnet, dass** die Rohrwand eine totraumfreie Innenfläche aufweist.

13. Verwendung der Polymerzusammensetzung nach mindestens einem der Ansprüche 1 bis 12 in einem Photobioreaktor, **dadurch gekennzeichnet, dass** die Rohrwand auf der Innenseite als ein statischer Mischer ausgebildet ist.

14. Verwendung der Polymerzusammensetzung nach mindestens einem der Ansprüche 1 bis 13 in einem Photobioreaktor, **dadurch gekennzeichnet, dass** die rohrförmigen Kunststoffformkörper mit einer Triclampverbindung verbindbar ausgebildet sind.

15. Verwendung der das Absorptionsverhalten modifizierenden Zusätze nach mindestens einem der Ansprüche 1 bis 14 in einem Photobioreaktor, **dadurch gekennzeichnet, dass** der Photobioreaktor aus mineralischem Glas oder Keramik ausgebildet ist.

## Geänderte Patentansprüche

### Geänderte Patentansprüche gemäss Regel 137(2) EPÜ.

**1.** Polymerzusammensetzung mit einem modifizierten Absorptions- und Transmissionsverhalten, insbesondere geeignet für dem Sonnenlicht oder geeigneten Kunstlichtquellen ausgesetzten Photobioreaktoren aus Kunststoffformkörpern, wobei in der Polymerzusammensetzung ein anorganischer oder organischer Reflektor zur Reflexion von Ultraviolettstrahlung vorgesehen ist, **dadurch gekennzeichnet, dass** das Polymer zusätzlich zu den herkömmlichen Standard-Additiven wahlweise eine oder eine Kombination der folgenden Substanzen aufweist:
- ein anorganischer oder organischer Nah-Infrarot-Absorber zur Absorption der langwelligen Strahlung,
- ein anorganischer oder organischer Reflektor zur Reflexion von sichtbarer, Nah-Infrarot- oder Infrarot-Strahlung,
- ein optischer Aufheller oder Fluoreszenzfarbstoff zur Umwandlung der absorbierten Ultraviolettstrahlung in sichtbares Licht oder Fluoreszenzlicht,
- ein photochromer Farbstoff zur lichtintensitätsabhängigen Modifizierung des Transmissionsverhaltens des Kunststoffformkörpers und
- ein antimikrobieller Zusatz zur Verhinderung oder Reduktion der organischen Ablagerungen in dem Photobioreaktor.

**2.** Polymerzusammensetzung nach dem Anspruch 1, **dadurch gekennzeichnet, dass** der Nah-Infrarot-Absorber vorzugsweise ein anorganisches auf seltenen Erdmetallen basierendes Pigment aufweist.

**3.** Polymerzusammensetzung nach mindestens einem der Ansprüche 1 oder 2, **dadurch gekennzeichnet, dass** der optische Aufheller vorzugsweise Verbindungen auf Thiophen-Benzoxazol-Basis aufweist.

**4.** Polymerzusammensetzung nach mindestens einem der Ansprüche 1 bis 3, **dadurch gekennzeichnet, dass** der photochrome Farbstoff vorzugsweise Spiro-Naphthoxazine oder Naphthopyrane aufweist.

**5.** Polymerzusammensetzung nach mindestens einem der Ansprüche 1 bis 4, **dadurch gekennzeichnet, dass** der antimikrobielle Zusatz vorzugsweise Verbindungen auf Carbamat- oder Silberbasis aufweist.

**6.** Polymerzusammensetzung nach mindestens einem der Ansprüche 1 bis 5, **dadurch gekennzeichnet, dass** das Polymer amorph oder teilkristallin ausgebildet ist.

**7.** Polymerzusammensetzung nach mindestens einem der Ansprüche 1 bis 6, **dadurch gekennzeichnet, dass** als Polymer wahlweise transparentes Polyvinylchlorid, Polycarbonat, Polymethylmetacrylat, Polyolefin, Polystyrol, Polyethylenterephthalat, Polybutylenterephthalat oder Kombinationen, teil- oder vollfluorierte Polymere, wie beispielsweise Polyvinylidenfluorid oder Perfluoralkoxalkan, Copolymeren oder Legierungen hieraus verwendet wird.

**8.** Verwendung der Polymerzusammensetzung nach mindestens einem der Ansprüche 1 bis 7 in einem als Photobioreaktor einsetzbaren Kunststoffformkörper, **dadurch gekennzeichnet, dass** die Wand des Photobioreaktors rohrförmig oder plattenförmig ausgebildet ist.

**9.** Verwendung der Polymerzusammensetzung nach mindestens einem der Ansprüche 1 bis 8 in einem Kunststoffformkörper, **dadurch gekennzeichnet, dass** der Kunststoffformkörper aus verschiedenen Schichten, beispielsweise durch Coextrusion, Kaschierung oder Beschichtung ausgebildet ist und dass die Zusätze in den verschiedenen Schichten unterschiedliche Konzentrationen aufweisen.

**10.** Verwendung der Polymerzusammensetzung nach mindestens einem der Ansprüche 1 bis 9 in einem Photobioreaktor, **dadurch gekennzeichnet, dass** der rohrförmig extrudierte Kunststoffformkörper eine Rohrwand mit einer schraubenlinienförmig ausgebildeten Innenfläche aufweist.

**11.** Verwendung der Polymerzusammensetzung nach mindestens einem der Ansprüche 1 bis 10 in einem Photobioreaktor, **dadurch gekennzeichnet, dass** die Rohrwand eine totraumfreie Innenfläche aufweist.

**12.** Verwendung der Polymerzusammensetzung nach mindestens einem der Ansprüche 1 bis 11 in einem Photobioreaktor, **dadurch gekennzeichnet, dass** die Rohrwand auf der Innenseite als ein statischer Mischer ausgebildet ist.

**13.** Verwendung der Polymerzusammensetzung nach mindestens einem der Ansprüche 1 bis 12 in einem Photobioreaktor, **dadurch gekennzeichnet, dass** die rohrförmigen Kunststoffformkörper mit einer Triclampverbindung verbindbar ausgebildet sind.

**14.** Verwendung der das Absorptionsverhalten modifizierenden Zusätze nach mindestens einem der Ansprüche 1 bis 13 in einem Photobioreaktor, **dadurch gekennzeichnet, dass** der Photobioreaktor aus mineralischem Glas oder Keramik ausgebildet ist.
